# EUROPEAN PATENT APPLICATION

(11) **EP 4 751 711 A1**
(43) Date of publication of application: **03.06.2026**
(21) Application number: 24872078.1
(22) Date of filing: 20.09.2024
(51) Int. Cl.: A61K 8/40, A61K 8/31, A61K 8/34, A61K 8/42, A61Q 5/00, A61Q 5/12

(54) **HAIR COSMETIC**

(30) Priority: 28.09.2023 JP 2023167722
(71) Applicant: NOF Corporation, Shibuya-ku Tokyo 150-6019 (JP)
(72) Inventor: YOSHIOKA Yuiko, Amagasaki-shi, Hyogo 660-0095 (JP); SAKATSUME Yuma, Amagasaki-shi, Hyogo 660-0095 (JP); MORIKAWA Toshiyuki, Amagasaki-shi, Hyogo 660-0095 (JP)
(74) Representative: Williams Powell
(86) International application number: PCT/JP2024/033627
(87) International publication number: WO 2025/070287

(57) **Abstract**

A hair cosmetic preparation includes, (A) from 0.1 to 12 mass% of an isoparaffin having an average carbon number from 50 to 250, (B) from 0.2 to 19 mass% of an isoparaffin having an average carbon number from 20 to 24, (C) from 0.5 to 5 mass% of at least one compound selected from the group consisting of a quaternary ammonium salt of Formula (1), and an aliphatic amidoamine of Formula (2) and a salt thereof, (D) from 1 to 10 mass% of an alcohol having from 14 to 24 carbon atoms, and water, and in the hair cosmetic preparation, the mass ratio of (A) to (B) ((A)/(B)) is from 1/20 to 3/2.

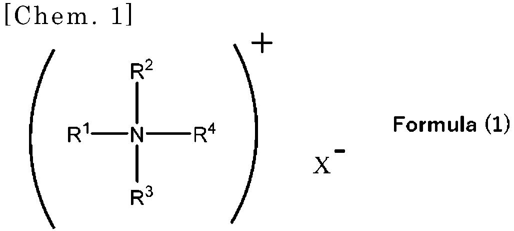

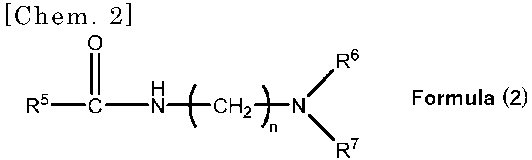

## Description

### Technical field

The present invention relates to a hair cosmetic preparation.

### Background Art

Hair is damaged every day by ultraviolet rays, chemicals in the air, washing and brushing, and heat from a hair dryer and a hair iron, etc. When damaged hair rubs against itself, the hair can become tangled, which impairs finger combability and/or hair alignment. Therefore, to improve finger combability and hair alignment, hair cosmetic preparations such as hair conditioners, hair treatments, hair rinses, hair packs, hair milks, and hair creams are used.

Hair cosmetic preparations typically contain a cationic surfactant that smoothens the hair surface, as well as an oily component such as hydrocarbon, an ester oil, an animal or vegetable oil, and/or silicone as appropriate. Among them, silicones are used in many hair cosmetic preparations because silicones have low surface tension and excellent lubricity and can impart excellent finger combability to dry hair.

For example, PTL 1 proposes a hair cosmetic preparation obtained by combining a liquid oil, a silicone, a specific oil or fat, and a polyhydric alcohol, and describes that the hair cosmetic preparation can impart, to hair, excellent finger combability after towel drying.

However, hair cosmetic preparations containing a large amount of silicone tend to make hair texture heavy with repeated use. In recent years, with diversification of consumer preferences, there has been an increasing demand for hair cosmetic preparation that contain a reduced amount of silicone and thus can provide a natural and light texture even after repeated use. The hair cosmetic preparation of PTL 1 may be inferior in terms of meeting this demand, i.e., maintaining the natural and light texture inherent in hair even after repeated use.

In addition, for example, diversification of lifestyles in recent years makes it difficult for consumers with long hair to care for their hair regularly, because hair care takes a considerable amount of time. Thus, there is a growing demand for sustaining excellent finger combability and hair alignment for the time between daily hair washes. To meet this demand, sustaining, from immediately after use, friction reducing effect is considered necessary, because friction coarsens hair and causes loss of hair alignment.

For example, PTL 2 proposes a silicone-free hair cosmetic preparation that contains a self-crosslinking compound and a compound having an IOB of 1.3 or less in the organic conceptual diagram and being liquid at 20°C. Although this hair cosmetic preparation excels in terms of finger combability during drying and suppression of stickiness after repeated use, the hair cosmetic preparation may be inferior in durability of the friction reducing effect due to its mild coating effect on the hair.

In this respect, PTL 3 proposes a hair cosmetic preparation containing a silicone, a cationic surfactant, and a higher alcohol. This hair cosmetic preparation is excellent in terms of durability of hair alignment and finger combability, that is, durability of the friction reducing effect.

However, the hair cosmetic preparation of PTL 3 may also be inferior in maintaining natural and light texture after repeated use.

Furthermore, in recent years, there has been an increasing demand for reducing time and effort for hair styling to tame a kink in hair, such as bed hair, in the morning. To meet this demand, it is necessary to realize both a strong coating effect on the hair surface for suppressing expansion of hair even when the hair is exposed to stress during sleep or the like, and an effect of imparting flexibility to the hair to suppress, even if the hair is expanded, kinking in hair, such as bed hair. However, the related-art technologies fail to achieve both of these effects, and may be inferior in hair expansion suppression effect that is an effect of maintaining alignment to suppress expansion of hair and suppressing kinking in hair, when the hair is exposed to stress.

### Citation List

### Patent Literature

PTL 1: JP 2017-193499 A
PTL 2: JP 2021-98688 A
PTL 3: JP 2021-143157 A

### Summary of Invention

### Technical Problem

In view of the above problems, an object of the present invention is to provide a hair cosmetic preparation that realizes excellent combability during drying, can provide a natural and light texture even after repeated use, realizes a long-lasting friction reducing effect on hair, and has an expansion suppression effect when the hair is exposed to stress.

### Solution to Problem

To solve the above problems, the inventor(s) conducted extensive research and found that a hair cosmetic preparation containing specific components described below can solve the above problems, and thus completed the present invention.

That is, the present invention a hair cosmetic preparation, including, (A) from 0.1 to 12 mass% of an isoparaffin having an average carbon number from 50 to 250, (B) from 0.2 to 19 mass% of an isoparaffin having an average carbon number from 20 to 24, (C) from 0.5 to 5 mass% of at least one compound selected from the group consisting of a quaternary ammonium salt of Formula (1), and an aliphatic amidoamine of Formula (2) and a salt thereof, (D) from 1 to 10 mass% of an alcohol having from 14 to 24 carbon atoms, and (E) water, and in the hair cosmetic preparation, the mass ratio of (A) to (B) ((A)/(B)) is from 1/20 to 3/2. 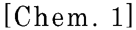 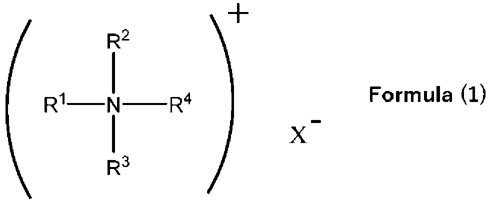 (In Formula (1), R¹ represents a hydrocarbon group having from 16 to 24 carbon atoms, a 2-hydroxy-3-alkyloxypropyl group having an alkyl chain with from 16 to 24 carbon atoms, or a 3-alkyloxypropyl group having an alkyl chain with from 16 to 24 carbon atoms, R² represents a hydrocarbon group having from 16 to 24 carbon atoms, an alkyl group having from 1 to 3 carbon atoms, or a hydroxyalkyl group having from 1 to 3 carbon atoms, R³ and R⁴ each independently represent an alkyl group having from 1 to 3 carbon atoms, or a hydroxyalkyl group having from 1 to 3 carbon atoms, and X represents a halogen atom.) 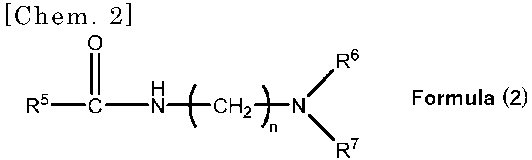 (In Formula (2), R⁵CO represents an acyl group having from 16 to 24 carbon atoms, R⁶ and R⁷ each independently represent an alkyl group having from 1 to 3 carbon atoms or a hydroxyalkyl group having from 1 to 3 carbon atoms, and n represents an integer from 1 to 3.)

### Advantageous Effects of Invention

The hair cosmetic preparation of the present invention realizes excellent combability during drying, can provide a natural and light texture even after repeated use, realizes a long-lasting friction reducing effect on hair, and has an expansion suppression effect when the hair is exposed to stress.

### Description of Embodiments

Hereinafter, embodiments of the present invention will be described. However, the present invention is not limited to the embodiments described herein, and various modifications are possible without departing from the spirit and scope of the present invention.

Note that, as used herein, any numerical range defined using "from ... to" is intended to include numerical values before and after "to" (the upper and lower limits). For example, "from 2 to 5" means 2 or more and 5 or less.

Furthermore, regarding any numerical range described herein, the upper or lower limit value of the numerical range can be replaced with a value presented in an example or a value that is uniquely derived from an example.

It is understood that values described herein are subject to the inherent variation characteristic of a measurement technique used to determine the values. In addition, numerical values should be interpreted in light of the number of significant digits and by applying rounding techniques.

A hair cosmetic preparation of the present invention contains the following Components (A), (B), (C), and (D). Each component will be explained below.

### [Component (A)]

Component (A) used in the present invention is an isoparaffin having an average carbon number from 50 to 250. This isoparaffin is a mixture of long-chain hydrocarbons having a side chain, and is typically obtained by hydrogenating a polymer made from isobutene and n-butene. Such components may include those generally classified as "heavy liquid isoparaffins" in the cosmetics field.

The average carbon number of the isoparaffin is from 50 to 250, preferably from 60 to 230, more preferably from 70 to 210, and even more preferably from 80 to 190. A too small average carbon number may result in poor expansion suppression effect when hair is exposed to stress. On the other hand, a too large average carbon number may result in poor combability during drying and/or difficulty in maintaining a natural and light texture.

The average carbon number in this specification is a value derived from peak area measurements and results of mass analysis obtained by using a gas chromatography-mass spectrometry (GC-MS) measuring device (JMS-T2000GC AccuTOF (registered trademark) GC-Alpha, manufactured by JEOL Ltd.).

Component (A) can be synthesized, for example, using the following method. First, a mixed gas of isobutene and n-butene can be processed using a known method, for example, cationic polymerization using a catalyst, to obtain a polymer. Then, the resulting polymer can be hydrogenated to obtain a hydrogenated polymer, which can be subjected to purification such as adsorption treatment and distillation to obtain Component (A).

Specific examples of Component (A) include "PARLEAM 18" (having an average carbon number of 72), "PARLEAM 24" (having an average carbon number of 96), and "PARLEAM 46" (having an average carbon number of 184), all manufactured by NOF CORPORATION. As Component (A), one or more of these isoparaffins having different average carbon numbers can be used.

### [Component (B)]

Component (B) used in the present invention is an isoparaffin having an average carbon number from 20 to 24, in other words, is a mixture of medium-chain hydrocarbons having a side chain, and is typically obtained by hydrogenating a polymer made from isobutene and n-butene. Such components may include those generally classified as "liquid isoparaffins" in the cosmetics field.

An average carbon number less than 20 increases volatility and may result in poor durability of friction reducing effect and/or poor expansion suppression effect when hair is exposed to stress. On the other hand, an average carbon number more than 24 may result in stickiness and inferiority in combability during drying and/or provision of a natural and light texture after repeated use.

Component (B) can be synthesized, for example, using the following method. First, a mixed gas of isobutene and n-butene can be processed using a known method, for example, cationic polymerization using a catalyst, to obtain a polymer. Then, the resulting polymer can be hydrogenated to obtain a hydrogenated polymer, which can be subjected to purification such as adsorption treatment and distillation to obtain Component (B).

Specific examples of Component (B) include "PARLEAM 6" (having an average carbon number of 24) and "PARLEAM EX" (having an average carbon number of 20) which are manufactured by NOF CORPORATION. As Component (B), one or more of these isoparaffins having different average carbon numbers can be used.

### [Component (C)]

Component (C) used in the present invention is at least one compound selected from the group consisting of a quaternary ammonium salt of Formula (1), and an aliphatic amidoamine of Formula (2) and a salt thereof.

The quaternary ammonium salt of Formula (1) will be explained.

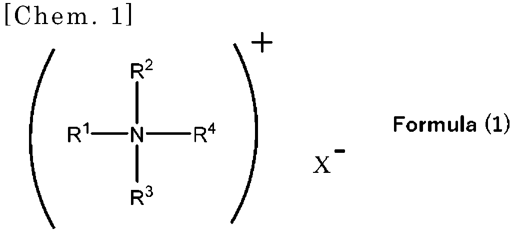

In Formula (1), R¹ represents a hydrocarbon group having from 16 to 24 carbon atoms, a 2-hydroxy-3-alkyloxypropyl group having an alkyl chain with from 16 to 24 carbon atoms, or a 3-alkyloxypropyl group having an alkyl chain with from 16 to 24 carbon atoms. The hydrocarbon group having from 16 to 24 carbon atoms may be a straight chain or branched, and may be saturated or unsaturated, but is preferably a straight-chain saturated hydrocarbon group, i.e., a straight-chain alkyl group. The alkyl chain in each of the 2-hydroxy-3-alkyloxypropyl group and the 3-alkyloxypropyl group may be a straight chain or branched, but is preferably a straight alkyl chain.

The hydrocarbon group and alkyl chain in R¹ preferably have from 18 to 22 carbon atoms. Specific examples of the hydrocarbon group (the alkyl chain) include a cetyl group, a stearyl group, an oleyl group, and a behenyl group. Among them, a cetyl group, a stearyl group, and a behenyl group are preferred.

R² represents a hydrocarbon group having from 16 to 24 carbon atoms, an alkyl group having from 1 to 3 carbon atoms, or a hydroxyalkyl group having from 1 to 3 carbon atoms. The hydrocarbon group having from 16 to 24 carbon atoms may be a straight chain or branched, and may be saturated or unsaturated, but is preferably a straight-chain saturated hydrocarbon group, i.e., a straight-chain alkyl group.

Preferable examples of R² include an alkyl group having from 1 to 3 carbon atoms and a hydroxyalkyl group having from 1 to 3 carbon atoms. Specific examples of the alkyl group include a methyl group, an ethyl group, and a propyl group, and specific examples of the hydroxyalkyl group include a hydroxymethyl group and a hydroxyethyl group.

R³ and R⁴ each independently represent an alkyl group having from 1 to 3 carbon atoms or a hydroxyalkyl group having from 1 to 3 carbon atoms. Specific examples of the alkyl group include a methyl group, an ethyl group, and a propyl group, and specific examples of the hydroxyalkyl group include a hydroxymethyl group and a hydroxyethyl group.

X represents a halogen atom, and specific examples thereof include chlorine, bromine, and iodine.

Next, the aliphatic amidoamine of Formula (2) and a salt thereof will be explained.

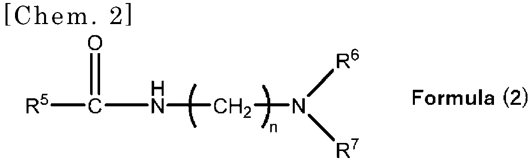

In Formula (2), R⁵CO represents an acyl group having from 16 to 24 carbon atoms. The acyl group having from 16 to 24 carbon atoms may be a straight chain or branched, and may be saturated or unsaturated. R⁵CO preferably has from 18 to 22 carbon atoms. Specific examples of the acyl group include a palmitoyl group, a stearoyl group, an oleoyl group, and a behenoyl group. Among them, a stearoyl group and a behenoyl group are preferred.

R⁶ and R⁷ each independently represent an alkyl group having from 1 to 3 carbon atoms or a hydroxyalkyl group having from 1 to 3 carbon atoms. Specific examples of the alkyl group include a methyl group, an ethyl group, and a propyl group, and specific examples of the hydroxyalkyl group include a hydroxymethyl group and a hydroxyethyl group.

n represents an integer from 1 to 3.

Examples of the salt of the aliphatic amidoamine of Formula (2) include hydrochloride salts and hydrobromide salts thereof.

Component (C) is preferably the quaternary ammonium salt of Formula (1). As Component (C), one or more compounds selected from the group consisting of the quaternary ammonium salt of Formula (1), and the aliphatic amidoamine of Formula (2) and a salt thereof can be used.

### [Component (D)]

Component (D) used in the present invention is an alcohol having from 14 to 24 carbon atoms. Examples of the hydrocarbon group of the alcohol include a straight-chain or branched-chain alkyl or alkenyl group, and preferably a straight-chain alkyl group.

Specific examples of Component (D) include alcohols such as myristyl alcohol, cetyl alcohol (cetanol), stearyl alcohol, arachidyl alcohol, behenyl alcohol, and tricosyl alcohol, as well as mixtures of these alcohols such as cetostearyl alcohol and hydrogenated rapeseed alcohol.

As Component (D), one or more of these alcohols and alcohol mixtures can be used, but a combination of two or more of the alcohols or alcohol mixtures is preferred, and a combination of one or more alcohols having from 14 to 16 carbon atoms with one or more alcohols having from 18 to 24 carbon atoms is more preferred. In particular, from the viewpoint of storage stability of the composition, it is preferable to combine alcohols so as to achieve an average carbon number from 16 to 22, and it is more preferable to combine alcohols so as to achieve an average carbon number from 16 to 18.

### [Content of Each Component]

In the present invention, the content of the above-mentioned Component (A) in the hair cosmetic preparation is from 0.1 to 12 mass%, preferably from 0.5 to 10 mass%, and more preferably from 1 to 8 mass%. A too low content of Component (A) may result in insufficiency in the expansion suppression effect when hair is exposed to stress. A too high content of Component (A) may result in insufficiency in combability during drying and/or maintaining a natural and light texture after repeated use.

In the present invention, the content of the above-mentioned Component (B) in the hair cosmetic preparation is from 0.2 to 19 mass%, preferably from 0.5 to 15 mass%, and more preferably from 2 to 12 mass%. A too small content of Component (B) may result in poor combability during drying and/or poor durability of the friction reducing effect. A too high content of Component (B) may result in insufficiency in the expansion suppression effect when, after repeated use, hair is exposed to stress, and may make it difficult to prepare a stable composition.

In the present invention, the content of the above-mentioned Component (C) in the hair cosmetic preparation is from 0.5 to 5 mass%, preferably from 0.8 to 4.5 mass%, and more preferably from 1 to 3 mass%. A too low content of Component (C) may result in insufficiency in combability during drying and/or durability of the friction reducing effect, and the expansion suppression effect when hair is exposed to stress. A too high content of Component (C) may result in insufficiency in maintaining a natural and light texture after repeated use.

In the present invention, the content of the above-mentioned Component (D) in the hair cosmetic preparation is from 1 to 10 mass%, preferably from 2 to 9 mass%, and more preferably from 3 to 8 mass%. A too low content of Component (D) may result in insufficiency in combability during drying and may make it difficult to prepare a stable composition. A too high content of Component (D) may result in insufficiency in a natural and light texture after repeated use and/or the expansion suppression effect when hair is exposed to stress.

In the present invention, the mass ratio of Component (A) to Component (B) ((A)/(B)) is from 1/20 to 3/2, preferably from 1/9 to 6/5, and more preferably from 1/4 to 1/1. A too low mass ratio ((A)/(B)) may result in insufficiency in the expansion suppression effect when hair is exposed to stress. A too high mass ratio ((A)/(B)) may result in insufficiency in combability during drying and/or maintaining a natural and light texture after repeated use.

### [Water]

The hair cosmetic preparation of the present invention contains water in addition to the above-described components (A) to (D). Examples of the water include purified water such as distilled water and ion-exchanged water, and tap water. Among them, purified water such as distilled water and ion-exchanged water is preferred.

The content of water in the hair cosmetic preparation is not particularly limited, and is from 50 to 95 mass%, preferably from 60 to 95 mass%, and more preferably from 65 to 90 mass%.

### [Other Components]

The hair cosmetic preparation of the present invention may contain an additive and/or a solvent as needed.

As additives, various components that are generally used in cosmetic preparations, quasi drugs, and pharmaceuticals can be blended as long as blending the additives does not impair the effects of the present invention. Examples of the additive include nonionic surfactants, higher fatty acids and triglycerides thereof, ester oils, oils and fats of animals and vegetables, hydrocarbon oils other than Component (A) and Component (B), silicones, quaternary ammonium salts and tertiary amines other than Component (C), vitamins, sequestering agents, thickeners, dyes, pigments, and fragrances.

Furthermore, as the solvent, ethanol, 1,3-butylene glycol, dipropylene glycol, propylene glycol, glycerin, etc. can be used as needed.

Examples of nonionic surfactants include polyoxyalkylene sorbitan fatty acid esters, polyoxyalkylene sorbit fatty acid esters, polyoxyalkylene glycerin fatty acid esters, polyoxyalkylene fatty acid esters, polyoxyalkylene alkyl ethers, polyoxyalkylene alkylphenyl ethers, polyoxyalkylene (hydrogenated) castor oils, sucrose fatty acid esters, polyglycerin alkyl ethers, polyglycerin fatty acid esters, fatty acid alkanolamides, and alkyl glucosides. Among them, alkyl glycosides, polyoxyalkylene C8-20 fatty acid esters, polyoxyethylene sorbitan fatty acid esters, polyoxyethylene hydrogenated castor oils, and fatty acid alkanolamides are preferred.

Examples of higher fatty acids include lauric acid, myristic acid, palmitic acid, stearic acid, behenic acid, isostearic acid, 12-hydroxystearic acid, oleic acid, and lanolin fatty acid.

Examples of the ester oil include an ester oil having in total from 8 to 40 carbon atoms, and preferably include an ester of a fatty acid having in total from 8 to 20 carbon atoms and an alcohol having from 1 to 20 carbon atoms. Among them, the ester oil is preferably isopropyl palmitate and isopropyl myristate.

Examples of oils and fats of animals and vegetables include horse oil, peony oil, deer fat, olive oil, camellia oil, shea butter, almond oil, safflower oil, sunflower oil, soybean oil, cottonseed oil, sesame oil, corn oil, rapeseed oil, rice bran oil, rice germ oil, grape seed oil, avocado oil, macadamia nut oil, castor oil, coconut oil, evening primrose oil, and cocoa butter.

Examples of hydrocarbon oils other than components (A) and (B) include squalane and a liquid paraffin.

Examples of silicones include cyclic silicones, methylpolysiloxanes, and amino-modified polysiloxanes.

Examples of quaternary ammonium salts and tertiary amines other than Component (C) include behentrimonium methosulfate.

Examples of vitamins include vitamin A and derivatives thereof; vitamins B such as vitamin B6 hydrochloride, vitamin B6 tripalmitate, vitamin B6 dioctanoate, vitamin B2 and derivatives thereof, vitamin B12, vitamin B15 and derivatives thereof, vitamin H, and pantothenic acid (pantethine); vitamins E such as α-tocopherol, β-tocopherol, γ-tocopherol, and vitamin E acetate; vitamins D; and pyrroloquinoline quinone.

Examples of sequestering agents include ethylenediaminetetraacetic acid (EDTA; edetic acid) and salts thereof, hydroxyethylethylenediaminetriacetic acid (HEDTA) and salts thereof, diethylenetriaminepentaacetic acid and salts thereof, and hydroxyethanediphosphonic acid (HEDP; etidronic acid) and salts thereof.

Examples of thickeners include guar gum, quince seed, carrageenan, galactan, gum arabic, pectin, mannan, starch, xanthan gum, curdlan, methyl cellulose, hydroxyethyl cellulose, carboxymethyl cellulose, methyl hydroxypropyl cellulose, chondroitin sulfate, dermatan sulfate, glycogen, heparan sulfate, hyaluronic acid, sodium hyaluronate, gum tragacanth, keratan sulfate, chondroitin, mucoitin sulfate, hydroxyethyl guar gum, carboxymethyl guar gum, dextran, locust bean gum, succinoglucan, colanic acid, chitin, chitosan, carboxymethyl chitin, agar, polyvinyl alcohol, polyvinylpyrrolidone, carboxyvinyl polymer, sodium polyacrylate, polyethylene glycol, and bentonite.

The content of the additive and/or solvent in the hair cosmetic preparation is not particularly limited, but is preferably 20 mass% or less, preferably 15 mass% or less, and more preferably 12 mass% or less.

The hair cosmetic preparation of the present invention can be prepared by using a known method, for example, by mixing the components while heating and then stirring the mixture. Note that, by appropriately adjusting the amount of water added, the content of each component can be adjusted to the desired level. The content of each of the above components is expressed as the content when the total content of all components of the hair cosmetic preparation is 100 mass%.

The hair cosmetic preparation of the present invention preferably has a pH of from 4 to 7, which may result in a strong effect as well as excellent stability over time. The pH can be adjusted using an organic acid such as citric acid, glutamic acid, or lactic acid, or a salt thereof.

The hair cosmetic preparation of the present invention can be used as a hair conditioner, hair treatment, hair rinse, hair pack, etc., each of which can be produced according to a conventional method.

### Examples

The present invention will be specifically explained below with reference to examples and comparative examples, but the present invention is not limited to these examples in any way.

### [Examples 1 to 10 and Comparative Examples 1 to 9]

### <Preparation of Hair Cosmetic Preparation>

Components (A), (B), and (D) shown in Tables 1 and 2 were mixed at 80°C, and the mixture was added to a mixture of Component (C) and water that had been mixed separately at 80°C. This mixture was mixed for 5 minutes using a high-speed homogenizer (HIGH FLEX DISPERSER HG92, SMT Co., Ltd.) to prepare hair cosmetic preparations of Examples 1 to 10 and Comparative Examples 1 to 9.

As the components shown in Tables 1 and 2, commercially available products for cosmetics were used. The product names of *1 to *7 in Tables 1 and 2 are listed below. Note that the content of each component shown in Tables 1 and 2 is in mass%.

*1: "PARLEAM 18" (NOF CORPORATION): Average carbon number, 72
*2: "PARLEAM 24" (NOF CORPORATION): Average carbon number, 96
*3: "PARLEAM 46" (NOF CORPORATION): Average carbon number, 184
*4: "PARLEAM 6" (NOF CORPORATION): Average carbon number, 24
*5: "PARLEAM EX" (NOF CORPORATION): Average carbon number, 20
*6: "KF-96-1000cs" (Shin-Etsu Chemical Co., Ltd.)
*7: "KF-96A-20cs" (Shin-Etsu Chemical Co., Ltd.)

The relationship between Components (C) in Tables 1 and 2 and the symbols in Formulas (1) and (2) is as follows:
C1: In Formula (1), R¹ is a behenyl group which is a straight-chain alkyl group having 22 carbon atoms, R², R³ and R⁴ are all methyl groups, and X is chlorine.
C2: In Formula (1), R¹ is a cetyl group which is a straight-chain alkyl group having 16 carbon atoms, R², R³ and R⁴ are all methyl groups, and X is chlorine.
C3: In Formula (1), R¹ is a 2-hydroxy-3-behenyloxypropyl group, R², R³ and R⁴ are all methyl groups, and X is chlorine.
C4: In Formula (2), R⁵CO is a stearoyl group which is a straight-chain acyl group having 18 carbon atoms, R⁶ and R⁷ are both methyl groups, and n is 3.

### <Evaluation of Hair Cosmetic Preparation>

### Hair Treatment Method

Healthy black hair (black human hair (root-aligned), item number: BS-B3A, 10 g, 30 cm, Beaulax Co., Ltd) was washed with 2 g of 25 mass% aqueous solution of sodium polyoxyethylene lauryl ether sulfate, thoroughly rinsed with tap water at 40°C, and then 2 g of each of the hair cosmetic preparations shown in Examples in Table 1 and Comparative Examples in Table 2 was applied to the hair. Two minutes after the application, the hair cosmetic preparation was rinsed out thoroughly with tap water at 40°C. After towel drying, the hair was dried with a dryer. The procedure from the washing with 25 mass% aqueous solution of sodium polyoxyethylene lauryl ether sulfate to the drying with the dryer was repeated 10 times, and then the hair was evaluated.

### (1) Combability During Drying

The hair was treated as described above, and combability during the 10th drying with the dryer was measured by passing a comb through the hair 15 times during the drying and counting the number of times the comb got caught on the hair. Based on the number of times the comb got caught, combability during drying was evaluated using a three grade scale ("Excellent", "Good", and "Marginal") according to the following evaluation criteria. "Excellent" and "Good" were considered to be acceptable.
Excellent: Once or less
Good: 2 or 3 times
Marginal: 4 times or more

### (2) Natural and Light Texture

The hair treated as described above was evaluated in terms of hair texture by six panelists (three men and three women, aged 25 to 45). By placing the hair between the thumb, index finger, and middle finger and sliding the fingers from the root to the tip, the texture was evaluated according to the following absolute evaluation criteria. The total score of evaluation results of the panelists was calculated, and based on the total score, the texture was evaluated using a three grade scale ("Excellent", "Good", and "Marginal") according to evaluation criteria described below. "Excellent" and "Good" were considered to be acceptable.

Note that panelists conducting the evaluations were trained in a preliminary evaluation test conducted prior to the evaluation tests such that the levels of evaluation corresponding to their respective scores were roughly the same, to equalize evaluation criteria of the panelists.

### <Absolute Evaluation Criteria>

### (Score): (Evaluation)

3: Natural and light texture
2: Slightly heavy texture
1: Heavy texture as if the hair is coated
0: The panelist felt that the hair is stiff and/or sticky.

### <Evaluation by Three Grade Scale Based on Total Score>

Excellent: Total score from 15 to 18 points
Good: Total score from 12 to 14 points
Marginal: Total score equal to or less than 11 points

### (3) Durability of Friction Reducing Effect

The coefficient of dynamic friction of the hair treated as described above was measured immediately after the 10th drying with the dryer and after 24 hours (left to stand in an environment of 23°C and 40% RH), by using a static/dynamic friction coefficient measuring device (multifunctional static/dynamic friction measuring device TL201Tt, Trinity-Lab. Inc.). For each measurement, three measurement values were acquired. The average values of the three measurement values were compared, and based on the difference between the average values immediately after the 10th drying with the dryer and after 24 hours, combability during drying was evaluated using a three grade scale ("Excellent", "Good", and "Marginal") according to the following evaluation criteria. "Excellent" and "Good" were considered to be acceptable.
Excellent: The difference between the average values is less than 0.20.
Good: The difference between the average values is 0.20 or more and less than 0.40.
Marginal: The difference between the average values is 0.40 or more.

### (4) Expansion Suppression Effect When Hair is Exposed to Stress

For the hair treated as described above, immediately after the 10th drying with the dryer, the expansion width of the hair at a location 15 cm above the tip of hair was measured. Thereafter, the hair was placed on a towel in a state of being waved, a towel was placed over the hair, and a 50 g plate was placed on top. This state was maintained for 24 hours. The bed hair artificially made in this manner was combed five times with a comb, and then the expansion width at a location 15 cm above the tip of hair was measured again. Based on the difference between the expansion width of the hair immediately after the 10th drying with the dryer and the expansion width of the bed hair that was made artificially, the expansion suppression effect when the hair is exposed to stress was evaluated using a three grade scale ("Excellent", "Good", and "Marginal") according to the following evaluation criteria. "Excellent" and "Good" were considered to be acceptable.
Excellent: The difference in the expansion width of the hair is less than 3.0 cm.
Good: The difference in the expansion width of the hair is 3.0 cm or more and less than 4.0 cm.
Marginal: The difference in the expansion width of the hair is 4.0 cm or more.

The hair cosmetic preparations of Examples 1 to 10 could provide excellent combability during drying, a natural and light texture even after repeated use, and a long-lasting friction reducing effect on hair, and had an expansion suppression effect when the hair was exposed to stress.

On the other hand, Comparative Example 1 did not contain Component (B), and thus exhibited poor combability during drying, failed to provide a natural and light texture, was inferior in durability of the friction reducing effect, and had a poor expansion suppression effect when the hair is exposed to stress.

Comparative Example 2 had a too large ratio (A)/(B), and thus exhibited poor combability during drying and failed to provide a natural and light texture.

Comparative Example 3 did not contain Component (A) and contained the silicones, and thus failed to provide a natural and light texture, and had a poor expansion suppression effect when the hair is exposed to stress.

Comparative Example 4 contained a large amount of Component (A) and had a too large ratio (A)/(B), and thus exhibited poor combability during drying and failed to provide a natural and light texture.

Comparative Example 5 contained an excessive amount of Component (C) and thus failed to provide a natural and light texture.

Comparative Example 6 contained a large amount of Component (B) and had a too small ratio (A)/(B), and thus had a poor expansion suppression effect when the hair is exposed to stress.

Comparative Example 7 did not contain Component (C), and thus exhibited poor combability during drying, was inferior in durability of the friction reducing effect, and had a poor expansion suppression effect when the hair is exposed to stress.

Comparative Example 8 did not contain Component (D), and thus exhibited poor combability during drying.

Comparative Example 9 contained an excessive amount of Component (D), and thus failed to provide a natural and light texture and had a poor expansion suppression effect when the hair is exposed to stress.

### [Table 1]

**Table 1**

| Component | | | Ex. 1 | Ex. 2 | Ex. 3 | Ex. 4 | Ex. 5 | Ex. 6 | Ex. 7 | Ex. 8 | Ex. 9 | Ex. 10 |
|---|---|---|---|---|---|---|---|---|---|---|---|---|
| Component (A) | A1 | Isoparaffin *1 | 9.0 | - | - | - | 12.0 | - | - | - | - | - |
| | A2 | Isoparaffin *2 | - | 1.5 | - | 1.4 | - | - | 1.0 | 2.0 | 0.6 | 1.5 |
| | A3 | Isoparaffin *3 | - | - | 0.2 | - | - | 0.5 | 0.5 | 1.0 | 0.2 | - |
| Component (B) | B1 | Isoparaffin *4 | 10.0 | 3.5 | - | - | 6.0 | 0.4 | 3.5 | 18.0 | 6.0 | 4.5 |
| | B2 | Isoparaffin *5 | 4.0 | - | 1.9 | 2.5 | 4.0 | - | - | - | - | - |
| Component (C) | C1 | Behentrimonium chloride | 0.5 | 2.0 | 2.0 | 0.7 | 2.0 | 2.0 | 4.8 | 3.0 | - | 0.5 |
| | C2 | Cetrimonium chloride | - | - | 1.0 | - | - | 1.5 | - | - | - | 1.5 |
| | C3 | Behenyl PG-trimonium chloride | 0.2 | - | - | - | - | - | - | - | - | - |
| | C4 | Stearamidopropyl dimethylamine | - | - | - | - | - | - | - | - | 2.5 | - |
| Component (D) | D1 | Behenyl alcohol (having 22 carbon atoms) | - | 0.3 | 1.5 | 0.1 | - | 0.4 | 0.4 | 0.4 | 0.5 | 3.5 |
| | D2 | Stearyl alcohol (having 18 carbon atoms) | 0.4 | 1.0 | 1.5 | 0.4 | 2.3 | 2.0 | 2.0 | 1.5 | 2.0 | 2.0 |
| | D3 | Cetanol (having 16 carbon atoms) | 0.8 | 2.7 | 6.5 | 2.0 | 6.7 | 4.6 | 5.6 | 4.1 | 4.2 | 0.5 |
| | Average carbon number of Component (D) | | 16.7 | 17.0 | 17.3 | 16.6 | 16.5 | 16.9 | 16.8 | 16.9 | 17.0 | 20.2 |
| Other components | Dimethylpolysiloxane *6 | | - | - | - | 1.7 | - | - | - | - | - | - |
| | Dimethylpolysiloxane *7 | | - | - | - | - | - | - | - | - | - | - |
| Water | Ion-exchanged water | | Balance | | | | | | | | | |
| Total | | | 100.0 | 100.0 | 100.0 | 100.0 | 100.0 | 100.0 | 100.0 | 100.0 | 100.0 | 100.0 |
| (A)/(B) | | | 9/14 | 3/7 | 2/19 | 14/25 | *6*/*5* | 5/4 | 3/7 | 1/6 | 2/15 | 1/3 |
| Evaluation | Combability during drying (times) | | Good (2) | Excellent (0) | Excellent (1) | Excellent (0) | Good (3) | Good (2) | Excellent (0) | Excellent (1) | Excellent (1) | Excellent (1) |
| | Natural and light tex:ure (score) | | Good (13) | Excellent (17) | Good (14) | Good (12) | Good (13) | Good (14) | Good (14) | Excellent (16) | Excellent (16) | Good (14) |
| | Durability of friction reducing effect | | Good (0.26) | Excellent (0.16) | Good (0.32) | Good (0.34) | Excellent (0.16) | Good (0.38) | Excellent (0.18) | Excellent (0.15) | Good (0.24) | Excellent (0.17) |
| | Expansion suppression effect when hair is exposed to stress (cm) | | Good (3.1) | Excellent (2.6) | Good (3.8) | Excellent (2.5) | Good (3.2) | Good (3.4) | Excellent (2.7) | Good (3.5) | Good (3.3) | Good (3.7) |

### [Table 2]

**Table 2**

| Component | | | Com. Ex. 1 | Com. Ex. 2 | Com. Ex. 3 | Com. Ex. 4 | Com. Ex. 5 | Com. Ex. 6 | Com. Ex. 7 | Com. Ex. 8 | Com. Ex. 9 |
|---|---|---|---|---|---|---|---|---|---|---|---|
| Component | A1 | Isoparaffin *1 | - | - | - | 15.0 | - | - | - | - | - |
| | A2 | Isoparaffin *2 | 5.0 | 10.0 | - | - | 1.0 | - | 1.0 | 1.0 | 1.0 |
| | A3 | Isoparaffin *3 | - | - | - | - | - | 0.6 | - | - | - |
| Component (B) | B1 | Isoparaffin *4 | - | 1.0 | - | 5.0 | 3.0 | | 3.0 | 3.0 | 1.0 |
| | B2 | Isoparaffin *5 | - | - | 1.5 | - | - | 23.0 | - | - | - |
| Component (C) | C1 | Behentrimonium chloride | 2.0 | 2.0 | 3.0 | 2.0 | 8.0 | 4.0 | - | 3.0 | 4.0 |
| | C2 | Cetrimonium chloride | - | - | - | - | - | - | - | - | - |
| | C3 | Behenyl PG-trimonium chloride | - | - | - | - | - | - | - | - | - |
| Component | D1 | Behenyl alcohol (having 22 carbon atoms) | 0.2 | 0.2 | 1.0 | 0.2 | 1.5 | 0.2 | - | - | - |
| | D2 | Stearyl alcohol (having 18 carbon atoms) | 1.5 | 1.5 | 2.0 | 1.5 | 1.5 | 1.5 | - | - | 22.0 |
| | D3 | Cetanol (having 16 carbon atoms) | 3.3 | 3.3 | 5.5 | 3.3 | 6.0 | 3.3 | 2.0 | - | - |
| | Average carbon number of Component (D) | | 16.8 | 16.8 | 17.2 | 16.8 | 17.3 | 16.8 | 16.0 | - | 18.0 |
| Other components | | Dimethylpolysiloxane *6 | - | - | 1.0 | - | - | - | - | - | - |
| | | Dimethylpolysiloxane *7 | - | - | 3.5 | - | - | - | - | - | - |
| Water | | Ion-exchanged water | Balance | | | | | | | | |
| Total | | | 100.0 | 100.0 | 100.0 | 100.0 | 100.0 | 100.0 | 100.0 | 100.0 | 100.0 |
| (A)/(B) | | | | 10/1 | 0 | 3/1 | 1/3 | 3/115 | 1/3 | 1/3 | 1/1 |
| Evaluation | | Combability during drying (times) | Marginal (10) | Marginal (8) | Excellent (0) | Marginal (6) | Excellent (0) | Excellent (1) | Marginal (5) | Marginal (4) | Good (2) |
| | | Natural and light texture (score) | Marginal (2) | Marginal (5) | Marginal (7) | Marginal (8) | Marginal (10) | Good (12) | Good (12) | Good (14) | Marginal (9) |
| | | Durability of friction reducing effect | Marginal (0.47) | Good (0.31) | Good (0.33) | Good (0.30) | Good (0.28) | Good (0.27) | Marginal (0.46) | Good (0.34) | Good (0.36) |
| | | Expansion suppression effect when hair is exposed to stress (cm) | Marginal (4.1) | Good (3.7) | Marginal (5.4) | Good (3.8) | Good (3.9) | Marginal (4.8) | Marginal (4.5) | Good (3.8) | Marginal (4.2) |

Next, example formulations of the hair cosmetic preparation of the present invention (Formulation Examples 1 and 2) are shown. The hair cosmetic preparations of these formulation examples were also evaluated according to the above (1) to (4). As a result, it was confirmed that the hair cosmetic preparations of the formulation examples could provide excellent combability during drying, a natural and light texture even after repeated use, and a long-lasting friction reducing effect on hair, and had an expansion suppression effect when the hair was exposed to stress.

In the following formulation examples, (A)/(B) is 3/7 for Formulation Example 1 and 1/4 for Formulation Example 2. In the following formulation examples, a component that is the same as any component in Tables 1 and 2 is designated by the same symbol.

### [Formulation Example 1: Conditioner]

| (Components) | (mass%) |
|---|---|
| 1. Isoparaffin (B1) with an average carbon number of 24 | 3.5 |
| 2. Ethylhexyl palmitate | 3.0 |
| 3. PPG-52 butyl ether | 3.0 |
| 4. Cetanol (D3) | 2.5 |
| 5. Glycerin | 2.0 |
| 6. Propylene glycol | 2.0 |
| 7. Isoparaffin (A2) with an average carbon number of 96 | 1.5 |
| 8. Behentrimonium chloride (C2) | 1.2 |
| 9. Stearyl alcohol (D2) | 1.0 |
| 10. Guar hydroxypropyltrimonium chloride | 0.5 |
| 11. Behenyl alcohol (D1) | 0.2 |
| 12. Polyquaternium-64 | 0.1 |
| 13. Citric acid | Proper amount |
| 14. Preservatives | Proper amount |
| 15. Fragrances | Proper amount |
| 16. Purified water | Balance |
| Total | 100.0 |

### [Formulation Example 2: Conditioner]

| (Components) | (mass%) |
|---|---|
| 1. Isoparaffin (B2) with an average carbon number of | 20 4.0 |
| 2. Propylene glycol | 4.0 |
| 3. Cetanol (D3) | 3.5 |
| 4. PPG-90 butyl ether | 3.0 |
| 5. Ethylhexyl palmitate | 3.0 |
| 6. Glycerin | 1.5 |
| 7. Isoparaffin (A3) with an average carbon number of 184 | 1.0 |
| 8. Behentrimonium chloride (C1) | 1.5 |
| 9. Stearyl alcohol (D2) | 1.5 |
| 10. Shea butter | 1.0 |
| 11. Beeswax | 0.5 |
| 12. Dimethicone | 0.4 |
| 13. Hydroxyethyl cellulose | 0.3 |
| 14. Behenyl alcohol (D1) | 0.3 |
| 15. PEG-75 stearate | 0.1 |
| 16. Citric acid | Proper amount |
| 17. Preservatives | Proper amount |
| 18. Fragrances | Proper amount |
| 19. Purified water | Balance |
| Total | 100.0 |

### [Industrial Applicability]

The hair cosmetic preparation of the present invention realizes excellent combability during drying, can provide a natural and light texture even after repeated use, realizes a long-lasting friction reducing effect on hair, and has an excellent expansion suppression effect when hair is exposed to stress. Thus, the hair cosmetic preparation of the present invention can be suitably used as a hair conditioner, hair treatment, hair rinse, hair pack, etc.

## Claims

1. A hair cosmetic preparation, comprising:
(A) from 0.1 to 12 mass% of an isoparaffin having an average carbon number from 50 to 250;
(B) from 0.2 to 19 mass% of an isoparaffin having an average carbon number from 20 to 24;
(C) from 0.5 to 5 mass% of at least one compound selected from the group consisting of a quaternary ammonium salt of Formula (1), and an aliphatic amidoamine of Formula (2) and a salt thereof;
(D) from 1 to 10 mass% of an alcohol having from 14 to 24 carbon atoms; and
water, wherein
the mass ratio of (A) to (B) ((A)/(B)) is from 1/20 to 3/2. 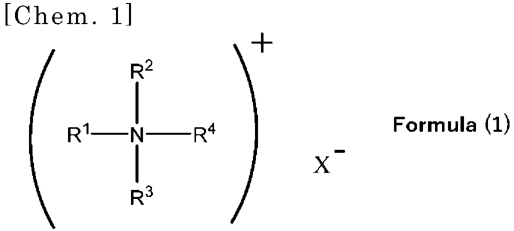
(In Formula (1), R¹ represents a hydrocarbon group having from 16 to 24 carbon atoms, a 2-hydroxy-3-alkyloxypropyl group having an alkyl chain with from 16 to 24 carbon atoms, or a 3-alkyloxypropyl group having an alkyl chain with from 16 to 24 carbon atoms, R² represents a hydrocarbon group having from 16 to 24 carbon atoms, an alkyl group having from 1 to 3 carbon atoms, or a hydroxyalkyl group having from 1 to 3 carbon atoms, R³ and R⁴ each independently represent an alkyl group having from 1 to 3 carbon atoms, or a hydroxyalkyl group having from 1 to 3 carbon atoms, and X represents a halogen atom.) 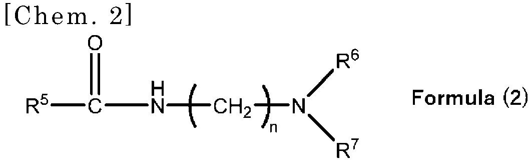 (In Formula (2), R⁵CO represents an acyl group having from 16 to 24 carbon atoms, R⁶ and R⁷ each independently represent an alkyl group having from 1 to 3 carbon atoms or a hydroxyalkyl group having from 1 to 3 carbon atoms, and n represents an integer from 1 to 3.)
